# EUROPÄISCHE PATENTSCHRIFT

(11) **EP 4 529 890 B1**
(45) Veröffentlichungstag und Bekanntmachung des Hinweises auf die Patenterteilung: **04.02.2026**
(21) Anmeldenummer: 23200305.3
(22) Anmeldetag: 28.09.2023
(51) Int. Cl.: A61F 2/40, A61L 27/16, A61L 27/54, A61B 17/56, A61F 2/30, A61F 2/46

(54) **INVERSER SCHULTERSPACER, GIESSFORM, KIT UND VERFAHREN ZUR HERSTELLUNG EINES INVERSEN SCHULTERSPACERS**
REVERSE SHOULDER SPACER, CASTING MOULD, KIT AND METHOD FOR PRODUCING AN INVERSE SHOULDER SPACER
ESPACEUR D'ÉPAULE INVERSE, MOULE, KIT ET PROCÉDÉ DE FABRICATION D'ESPACEUR D'ÉPAULE INVERSE

(43) Veröffentlichungstag der Anmeldung: 02.04.2025
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(56) Entgegenhaltungen:
- EP-A1- 3 906 896
- US-A1- 2017 157 579
- US-A1- 2022 015 911
- US-A1- 2022 192 835
- US-A1- 2022 296 381

## Beschreibung

Die Erfindung gemäß Anspruch 1 betrifft einen inversen Schulterspacer zum zeitweisen Ersetzen eines künstlichen Schultergelenks, umfassend
eine Glenoidkomponente, welche an einem distalen Glenoidkomponentenende als eine konvexe Halbkugel ausgebildet ist,
eine Humeruskomponente, welche an einem proximalen Humeruskomponentenende eine konkave Kugelaufnahme zur Aufnahme der Halbkugel der Glenoidkomponente aufweist, wobei die Halbkugel mit der Kugelaufnahme ein inverses Schultergelenk nachbildet oder nachbilden kann.

Die Erfindung betrifft weiterhin eine Gießform gemäß Anspruch 8 sowie ein Kit gemäß Anspruch 13 umfassend eine derartige Gießform und ein Verfahren gemäß Anspruch 14 zur Herstellung eines derartigen Schulterspacers.

### Hintergrund der Erfindung

Gelenkendoprothesen, wie beispielsweise Schulterprothesen, haben gegenwärtig eine Standzeit von mehreren Jahren, zum Beispiel bei zementierten Hüftgelenkprothesen im Durchschnitt von zehn bis fünfzehn Jahren. Es kann jedoch zu unerwünschten Lockerungen der Gelenkendoprothesen kommen, welche bereits vor dem Erreichen der üblichen Standzeiten auftreten. Dabei wird zwischen einer septischen und einer aseptischen Lockerung unterschieden. Bei der aseptischen Lockerung lassen sich bisher keine mikrobiellen Keime nachweisen. Die Ursachen der aseptischen Lockerungen können vielfältig sein. Häufig sind aseptische Lockerungen auf Abrieb an den Gleitflächen der Gelenkendoprothesen zurückzuführen.

Bei der septischen Lockerung wird der Lockerungsprozess durch mikrobielle Keime hervorgerufen. Man unterscheidet dabei in Abhängigkeit vom zeitlichen Auftreten frühe und späte Infektionen. Die septische Lockerung ist eine sehr ernste Erkrankung für den Patienten, die zusätzlich bei der Behandlung mit sehr hohen Kosten verbunden ist. Sowohl bei der aseptischen als auch der septischen Lockerung wird üblicherweise eine Revision vorgenommen. Man unterscheidet einzeitige und zweizeitige Revisionen. Bei septischen Lockerungen werden sehr häufig zweizeitige Revisionen vorgenommen.

Bei der zweizeitigen Revision wird in einer ersten Operation (OP) die infizierte Gelenkendoprothese entfernt und infiziertes Gewebe in einem sogenannten Debridement entfernt. Anschließend wird ein temporärer Platzhalter, ein so genannter Spacer, eingesetzt. Dieser Spacer füllt für einige Wochen den Raum der zuvor revidierten Gelenkendoprothese aus, bis die vorliegende Infektion abgeklungen ist. Diese Platzhalterfunktion ist sehr wichtig, um eine Atrophie der Muskulatur in dieser Zeit wirksam zu verhindern und um eine Stabilisierung der Resektionssituation zu erreichen.

Man unterscheidet nichtartikulierende und artikulierende Spacer. Artikulierende Spacer bilden die Gelenkfunktion nach und erlauben eine gewisse Beweglichkeit der betroffenen Gliedmaßen. Es ist dadurch möglich, die Patienten frühzeitig zu mobilisieren. Artikulierende Spacer werden daher derzeit besonders gerne eingesetzt. Als artikulierende Spacer kommen vor allem Hüftspacer, Kniespacer, Ellenbogenspacer oder Schulterspacer zum Einsatz. Der Spacer wird in einer zweiten OP entfernt, es wird nochmals debridiert und anschließend eine zementierte oder auch zementfreie Revisions-Gelenkendoprothese implantiert.

Spacer aus Polymethylmethacrylat-Knochenzement sind als temporäre Platzhalter für zweizeitige septische Revisionen von künstlichen Hüft-, Knie- und Schultergelenken allgemeiner Stand der Technik. Exemplarisch sind dafür folgende Patentschriften: US 6,361,731B1, US 7,637,729 B2, US 7,789,646 B2, US 8,480,389 B2 und US 8,801,983 B2, US 7,637,729 B2, US 10,071,511 B2, EP 2651324 B1 und EP 2526900 B1. Üblicherweise enthalten die Spacer ein Antibiotikum oder mehrere Antibiotika, die nach der Implantation der Spacer durch Einwirkung von Körperflüssigkeit aus der Oberfläche der Spacer herausgelöst werden und einen antibiotischen Schutz der Spaceroberfläche bewirken.

Die US 2022/015911 A1 offenbart einen Humerusspacer, welcher an seinem proximalen Ende eine konvexe Halbkugel aufweist und somit für einen anatomischen Schulterspacer vorgesehen ist. Zudem wird eine entsprechende Gießform sowie ein Kit und ein Verfahren zur Herstellung des Humerusspacers offenbart.

Im Bereich der artikulierenden Schulterspacer bilden einfache künstliche Schultergelenkprothese die natürliche anatomische Situation des Schultergelenks nach und ersetzen den Halbkugelkopf am proximalen Humerus durch eine geeignete Halbkugel, wobei diese durch einen Schaft im Knochenkanal des Humerus fixiert ist. Im gegenüberliegenden Glenoid wird eine Halbschale zur Aufnahme des Halbkugelkopfes fixiert.

Es gibt jedoch Fälle, bei denen beispielsweise die Rotatorenmanschette, der proximale Humerus und/oder das Glenoid derart stark geschädigt ist, dass eine derartige einfache Schultergelenkprothese nicht mehr sinnvoll implantiert werden kann. In diesen Fällen werden häufig sogenannte inverse Schulterprothesen eingesetzt.

Dabei wird eine Platte auf das Glenoid geschraubt, auf die wiederum eine konvexe Halbkugel als Gleitfläche angeordnet wird. Im Humerus wird als Gegenstück ein Schaft implantiert, der an seinem proximalen Ende eine konkave halbkugelförmige Aufnahme für die Halbkugel besitzt. Die konvexe Halbkugel und die konkave halbkugelförmige Aufnahme greifen ineinander und bilden ein künstliches Gelenk, das invers zur normalen anatomischen Situation aufgebaut ist. Es gibt eine Vielzahl von Herstellern inverser Schulterprothesen. Diese Prothesen unterscheiden sich unter anderem dadurch, dass die am Glenoid angeschraubte Prothesenkomponente unterschiedlich gestaltete Verankerungslöcher für Bolzen oder Schrauben aufweisen, um diese am Glenoid zu befestigen. Die Verankerungslöcher sind im Allgemeinen so gestaltet, dass eine Befestigung in verschiedenen Winkeln möglich ist, um bei der jeweiligen anatomischen Situation des Glenoids der Patienten eine sichere Verankerung der Glenoidkomponente zu gewährleisten.

Bisher sind jedoch noch keine inversen Schulterspacer bekannt.

### Aufgaben

Eine Aufgabe der vorliegenden Erfindung ist es, einen oder mehrere der sich aus dem Stand der Technik ergebenen Nachteile zumindest teilweise zu überwinden.

Im Speziellen basiert die Erfindung auf dem Ziel, einen inversen Schulterspacer zur Verfügung zu stellen. Der Schulterspacer soll möglichst einfach aufgebaut sein und für eine Vielzahl unterschiedlicher inverser Schulterprothesen als zeitweiser Ersatz verwendbar sein. Insbesondere soll der Schulterspacer temporär implantierbar sein, ohne das zusätzliche Verankerungslöcher im Glenoid eines Patienten geschaffen werden müssen.

Es ist eine weitere Aufgabe der Erfindung, eine Gießform und ein Kit zur Herstellung eines inversen Schulterpacers bereitzustellen, mittels dem mindestens ein Teil der bereits beschriebenen Aufgaben zumindest zum Teil gelöst wird.

Weiterhin besteht eine Aufgabe der Erfindung darin, ein Verfahren zur Herstellung eines inversen Schulterspacers bereitzustellen, mittels dem mindestens ein Teil der bereits beschriebenen Aufgaben zumindest zum Teil gelöst wird.

### Bevorzugte Ausführungsformen der Erfindung

Ein Beitrag zur mindestens teilweisen Erfüllung mindestens einer der zuvor genannten Aufgaben wird durch die Merkmale der unabhängigen Ansprüche geleistet. Die abhängigen Ansprüche stellen bevorzugte Ausführungsformen bereit, die zur mindestens teilweisen Erfüllung mindestens einer der Aufgaben beitragen.

Eine erste Ausführungsform der Erfindung ist ein inverser Schulterspacer zum zeitweisen Ersetzen eines künstlichen Schultergelenks, umfassend eine Glenoidkomponente, welche an einem distalen Glenoidkomponentenende als eine konvexe Halbkugel ausgebildet ist, eine Humeruskomponente, welche an einem proximalen Humeruskomponentenende eine konkave Kugelaufnahme zur Aufnahme der Halbkugel der Glenoidkomponente aufweist, wobei die Halbkugel mit der Kugelaufnahme ein inverses Schultergelenk nachbildet oder nachbilden kann,
dadurch gekennzeichnet, dass
der Schulterspacer einen oder mehrere Stifte aufweist, welche jeweils mit einem Stiftende mit einer von einer Vielzahl an konkaven Stiftaufnahmen an einem proximalen Glenoidkomponentenende der Glenoidkomponente verbunden oder verbindbar sind, so dass der Schulterspacer über den oder die Stifte in Verankerungslöchern in einem Glenoid eines Patienten befestigbar ist.

In einer Ausführungsform des Schulterspacers umfasst die Humeruskomponente und die Glenoidkomponente einen Polymethylmethacrylat-Knochenzement (PMMA-Knochenzement) oder die Humeruskomponente und die Glenoidkomponente bestehen aus einem Polymethylmethacrylat-Knochenzement. Diese Ausführungsform ist eine zweite Ausführungsform der Erfindung, welche vorzugsweise von der ersten Ausführungsform der Erfindung abhängt.

In einer Ausführungsform des Schulterspacers sind die Stiftaufnahmen konusartig ausgebildet, wobei sich ein Innenumfang der Stiftaufnahmen von einer Stiftaufnahmenöffnung in Richtung des distalen Glenoidkomponentenendes verfüngt. Diese Ausführungsform ist eine dritte Ausführungsform der Erfindung, welche vorzugsweise von der ersten oder der zweiten Ausführungsform der Erfindung abhängt.

In einer Ausführungsform des Schulterspacers weisen die konusartigen Stiftaufnahmen eine Mantelfläche auf, welche mit einer Konuslängsachse der Stiftaufnahmen einen Winkel in einem Bereich von 10-30 °, vorzugsweise in einem Bereich von 15-30 °, weiter bevorzugt in einem Bereich von 20-30 ° einschließt. Diese Ausführungsform ist eine vierte Ausführungsform der Erfindung, welche vorzugsweise von der dritten Ausführungsform der Erfindung abhängt.

In einer Ausführungsform des Schulterspacers weisen die Stifte, sofern mehr als ein Stift vorhanden ist, einen unterschiedlichen Durchmesser, insbesondere unterschiedlichen durchschnittlichen Durchmesser, eine unterschiedliche Länge oder einen unterschiedlichen Durchmesser, insbesondere unterschiedlichen durchschnittlichen Durchmesser und eine unterschiedliche Länge auf. Diese Ausführungsform ist eine fünfte Ausführungsform der Erfindung, welche vorzugsweise von einer der vorherigen Ausführungsformen der Erfindung abhängt.

In einer Ausführungsform des Schulterpacers weisen der oder die Stifte einen Stiftmetallkern auf. Diese Ausführungsform ist eine sechste Ausführungsform der Erfindung, welche vorzugsweise von einer der vorherigen Ausführungsformen der Erfindung abhängt.

In einer Ausführungsform des Schulterspacers weist die Humeruskomponente einen Humeruskomponentenmetallkern auf. Diese Ausführungsform ist eine siebte Ausführungsform der Erfindung, welche vorzugsweise von einer der vorherigen Ausführungsformen der Erfindung abhängt.

Eine achte Ausführungsform der Erfindung ist eine Gießform zur Herstellung eines inversen Schulterspacers nach einer der vorhergehenden Ausführungsformen der Erfindung, umfassend eine aus mindestens zwei Teilen bestehende Humerusteilgießform zur Ausbildung der Humeruskomponente,
eine aus mindestens zwei Teilen bestehende Glenoidteilgießform zur Ausbildung der Glenoidkomponente und
eine aus mindestens zwei Teilen bestehende Stiftteilgießform zur Ausbildung des Stiftes oder der Stifte.

In einer Ausführungsform umfasst die Gießform eine Vielzahl an Stiftteilgießformen mit einem unterschiedlichen Stiftteilgießformdurchmesser, einer unterschiedlichen Stiftteilgießformlänge oder einem unterschiedlichen Stiftteilgießformdurchmesser und einer unterschiedlichen Stiftteilgießformlänge. Diese Ausführungsform ist eine neunte Ausführungsform der Erfindung, welche vorzugsweise von der achten Ausführungsformen der Erfindung abhängt.

In einer Ausführungsform der Gießform umfasst die Humerusteilgießform Befestigungsmittel zum Einlegen eines Humeruskomponentenmetallkerns. Diese Ausführungsform ist eine zehnte Ausführungsform der Erfindung, welche vorzugsweise von der achten oder neunten Ausführungsform der Erfindung abhängt.

In einer Ausführungsform der Gießform bestehen die Teilgießformen aus einem transluzenten Polymer. Diese Ausführungsform ist eine elfte Ausführungsform der Erfindung, welche vorzugsweise von einer der achten bis zehnten Ausführungsform der Erfindung abhängt.

In einer Ausführungsform der Gießform sind die einzelnen Teile, oder zumindest zwei der einzelnen Teile, der Teilgießform durch Rastelemente reversibel lösbar miteinander verbindbar. Diese Ausführungsform ist eine zwölfte Ausführungsform der Erfindung, welche vorzugsweise von einer der achten bis elften Ausführungsform der Erfindung abhängt.

Eine dreizehnte Ausführungsform der Erfindung ist ein Kit zur Herstellung eines inversen Schulterspacers nach einer der ersten bis siebten Ausführungsformen der Erfindung umfassend eine Gießform nach einer der achten bis zwölften Ausführungsform der Erfindung sowie ein Knochenzementpulver und eine Monomerflüssigkeit zum Bereitstellen eines Polymethylmethacrylat-Knochenzementteigs (PMMA-Knochenzementteig).

Eine vierzehnte Ausführungsform der Erfindung ist ein Verfahren zur Herstellung eines inversen Schulterspacers nach einer der ersten bis siebten Ausführungsform der Erfindung mittels eines Kits nach der dreizehnten Ausführungsform der Erfindung, umfassend die folgenden Verfahrensschritte:
a. Bereitstellen eines Polymethylmethacrylat-Knochenzementteigs durch Anmischen des Knochenzementpulvers mit der Monomerflüssigkeit;
b. Befüllen der Humerusteilgießform, der Glenoidteilgießform und der Stiftteilgießform mit dem Polymethylmethacrylat-Knochenzementteig;
c. Aushärten des Polymethylmethacrylat-Knochenzementteigs unter Bereitstellung der Humeruskomponente, der Glenoidkomponente und des Stiftes.

In einer Ausführungsform des Verfahrens findet in einem Verfahrensschritt d. ein Befestigen des Stiftes in einer der Stiftaufnahmen der Glenoidkomponente statt. Diese Ausführungsform ist eine fünfzehnte Ausführungsform der Erfindung, welche vorzugsweise von der vierzehnten Ausführungsform der Erfindung abhängt.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Wert Y und kleiner als Y.

Einige der beschriebenen Merkmale sind mit dem Begriff "im Wesentlichen" verknüpft. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "Parallelität" nie exakt, sondern nur innerhalb gewisser fertigungstechnischer Fehlertoleranzen gegeben sein kann. Beispielsweise schließen "im Wesentlichen senkrechte Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%. Eine "im Wesentlichen vollständige Befüllung eines Volumens B" umfasst beispielsweise eine Befüllung von ≥95 bis ≤100 Volumen-% des Gesamtvolumens von B.

Begriffe wie "proximal" und "distal" dienen lediglich der Bezeichnung der räumlich gegenüberliegenden Enden der Vorrichtung oder anderer Struktureinheiten der Vorrichtung und erlauben keinen Rückschluss auf die Orientierung in Bezug zu einem menschlichen Körper, beispielsweise eines Anwenders der Vorrichtung. "Distal zu ..." und "proximal zu..." oder ähnliche Formulierungen drücken entsprechend lediglich die räumliche Anordnung zweier Struktureinheiten der Vorrichtung zueinander aus.

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet. Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

### Ausführliche Beschreibung

Ein erster Gegenstand der Erfindung betrifft einen inversen Schulterspacer, insbesondere einen artikulierenden inversen Schulterspacer, zum zeitweisen Ersetzen eines künstlichen Schultergelenks, umfassend eine Glenoidkomponente, welche an einem distalen Glenoidkomponentenende als eine konvexe Halbkugel ausgebildet ist,
eine Humeruskomponente, welche an einem proximalen Humeruskomponentenende eine konkave Kugelaufnahme zur Aufnahme der Halbkugel der Glenoidkomponente aufweist, wobei die Halbkugel mit der Kugelaufnahme ein inverses Schultergelenk nachbildet oder nachbilden kann,
dadurch gekennzeichnet, dass
der Schulterspacer einen oder mehrere Stifte aufweist, welche jeweils mit einem Stiftende mit einer von einer Vielzahl an konkaven Stiftaufnahmen an einem proximalen Glenoidkomponentenende der Glenoidkomponente verbunden oder verbindbar sind, so dass der Schulterspacer über den oder die Stifte in Verankerungslöchern in einem Glenoid eines Patienten befestigbar ist.

Der Schulterspacer umfasst eine Glenoidkomponente, welche zur Befestigung an einem Glenoid eines Patienten bestimmt ist. Die Glenoidkomponente ist an einem distalen Glenoidkomponentenende, dies entspricht vorzugsweise im implantierten Zustand dasjenige Ende der Glenoidkomponente, welches nicht direkt am Glenoid befestigt ist, sondern sich in Richtung des Humerus des Patienten erstreckt, als eine konvexe Halbkugel ausgebildet. In einer Ausführungsform sind die Halbkugel und das am Glenoid des Patienten befestigte proximale Glenoidkomponentenende einteilig ausgestaltet. In einer weiteren Ausführungsform kann das proximale Glenoidkomponentenende als eine Basis ausgeformt sein, auf welchem die konvexe Halbkugel befestigt, beispielsweise geschraubt oder geklebt, ist.

Der Schulterspacer umfasst weiterhin eine Humeruskomponente. Die Humeruskomponente weist an einem proximalen Humeruskomponentenende, dies entspricht im implantierten Zustand das dem Glenoid des Patienten zugewandten Ende der Humeruskomponente, eine konkave Kugelaufnahme zur Aufnahme der Halbkugel der Glenoidkomponente auf. Dabei entspricht vorzugsweise ein Außendurchmesser der Halbkugel im Wesentlichen einem Innendurchmesser der Kugelaufnahme, so dass die Halbkugel und die Kugelaufnahme im implantierten Zustand zusammenwirken, also die Halbkugel zumindest anteilig in die Kugelaufnahme aufgenommen ist, und so gemeinsam ein inverses Schultergelenk nachbilden. Die Halbkugel bildet somit im implantierten Zustand des Schulterspacers eine Gleitfläche mit der Kugelaufnahme der Humeruskomponente als Gegenstück. Vorzugsweise weist die Humeruskomponente an einem, dem proximalen Humeruskomponentenende gegenüberliegenden, distalen Humeruskomponentenende einen Schaft auf, welcher im Knochenkanal des Humerus des Patienten befestigbar ist, um die Humeruskomponente sicher mit diesem zu verbinden.

Um die Glenoidkomponente mit einem Glenoid eines Patienten zu verbinden, weist der Schulterspacer einen oder mehrere, beispielsweise zwei, drei, vier oder fünf, Stifte auf. Die Stifte sind ausgestaltet und bestimmt, in bereits vorhandene Verankerungslöcher im Glenoid des Patienten mit einem Stiftende eingeführt und dort, vorzugsweise mittels eines Polymethylmethacrylat-Knochenzements, befestigt zu werden. Vorzugsweise werden die gleichen Verankerungslöcher zum Befestigen der Glenoidkomponente benutzt, welche bereits zur Befestigung der primären Schulterprothese zum Einsatz kamen. Somit sind keine zusätzlichen Verankerungslöcher notwendig, was sowohl den Aufwand reduziert als auch möglichst viel Knochensubstanz des Patienten im Zuge der Revision der Schulterprothese erhält.

Mit dem Stiftende, welches nicht zum Einführen in die Verankerungslöcher im Glenoid des Patienten bestimmt ist, sind der oder die Stifte mit der Glenoidkomponente verbindbar oder, zumindest im implantierten Zustand des Schulterspacers, verbunden. Dazu weist die Glenoidkomponente am proximalen Glenoidkomponentenende, dies entspricht im implantierten Zustand das dem Glenoid des Patienten zugewandte Ende der Glenoidkomponente, eine Vielzahl, also mindestens zwei, beispielsweise sechs bis dreißig, vorzugsweise zehn bis zwanzig, weiter bevorzugt zwölf bis achtzehn, konkave Stiftaufnahmen auf, in welche die Stifte mit dem Stiftende einführbar und in den Stiftaufnahmen befestigt oder befestigbar sind. Die Stiftaufnahmen bilden somit konkave Vertiefungen am proximalen Glenoidkomponentenende, welche zum sicheren Befestigen der Stifte nutzbar sind. Eine Befestigung in den Stiftaufnahmen ist oder wird vorzugsweise mittels eines Polymethylmethacrylat-Knochenzements bewerkstelligt. Die Vielzahl an konkaven Stiftaufnahmen erlaubt eine vielseitige und flexible Befestigung der Stifte, je nach Lage der im Glenoid des Patienten vorhandenen Verankerungslöcher. Die Stiftaufnahmen sind vorzugsweise gleichmäßig über die, vorzugsweise gesamte, Fläche des proximalen Glenoidkomponentenendes verteilt. Somit kann für unterschiedliche am im Markt erhältlichen oder zukünftig erhältlichen Modell von inversen Schulterprothesen der gleiche erfindungsgemäße inverse Schulterspacer zum Einsatz kommen.

Der Schulterspacer kann aus unterschiedlichen Materialien oder Materialkombination gefertigt sein.

Eine Ausführungsform des Schulterspacers ist dadurch gekennzeichnet, dass zumindest die Humeruskomponente und die Glenoidkomponente einen Polymethylmethacrylat-Knochenzement umfassen oder aus einem Polymethylmethacrylat bestehen. Polymethylmethacrylat-Knochenzemente haben sich für Spacer für Knie und Hüften bewährt, sind kostengünstig und können mit einem oder mehreren Antibiotika bestückt werden, was sich vorteilhaft auf die Verwendung als Spacer auswirkt. Zudem erlaubt die Verwendung von Polymethylmethacrylat-Knochenzement als Material für den Schulterspacers eine einfache und schnelle Herstellung des Schulterspacers auch im Zuge einer laufenden Operation. Gleichzeitig mit der Herstellung kann die Zugabe eines oder mehrerer Antibiotika zum Polymethylmethacrylat durch den Operateur erwogen werden.

Vorzugsweise bilden die Stiftaufnahmen zumindest ungefähr einen Querschnitt der Stifte nach, so dass eine Befestigung der Stifte einfach, schnell und sicher möglich ist. Da die Verankerungslöcher üblicherweise einen runden Querschnitt aufweisen, weisen vorzugsweise auch die Stifte und somit auch die Verankerungslöcher einen runden Querschnitt auf. Beispielsweise sind sowohl die Stifte als auch die Verankerungslöcher zylinderartig ausgestaltet.

Eine Ausführungsform des Schulterspacers ist dadurch gekennzeichnet, dass die Stiftaufnahmen konusartig ausgebildet sind, wobei sich einen Innenumfang der Stiftaufnahmen von einer Stiftaufnahmenöffnung in Richtung des distalen Glenoidkomponentenendes verjüngt. Die Stiftaufnahmen sind in dieser Ausführungsform also trichterartig ausgestaltet, wobei die Stiftaufnahmenöffnung, in welche die Stifte einführbar sind, den größten Innenumfang, in anderen Worten den größten Innendurchmesser, aufweisen, wobei sich der Innenumfang immer weiter verjüngt, also kleiner wird, je weiter sich die Stiftaufnahme in Richtung der Halbkugel erstreckt. Diese Ausgestaltung erlaubt eine gewisse Variation einer Ausrichtung der Stifte im Verhältnis zu einer Längsachse der Glenoidkomponente. So können die an der Glenoidkomponente befestigten Stifte besser die tatsächlich vorhandenen Verankerungslöcher im Glenoid des Patienten angepasst werden. Beispielsweise kann die Ausrichtung der Stifte somit besser an eine Situation abgestimmt werden, in welcher die Verankerungslöcher im Glenoid des Patienten nicht alle parallel zueinander laufende Längsachsen aufweisen. Dabei können die Stiftaufnahmen im Wesentlichen die Form eines Konus oder eines Konusstumpfes aufweisen.

Die konusartigen Stiftaufnahmen dieser Ausführungsform können unterschiedlich steile Mantelflächen, in anderen Worten Seitenwände, aufweisen, was sich auf die Variabilität beim Ausrichten der darin befestigten Stifte im Verhältnis zur Längsachse der Glenoidkomponente auswirkt. Je größer ein Winkel, welcher zwischen einer Konuslängsachse und der Mantelfläche der konusartigen Stiftaufnahme eingeschlossen wird, desto variabler ist die Ausrichtung der Stifte. Gleichzeitig wird die sichere Befestigung der Stifte in den Stiftaufnahmen mit wachsendem Winkel erschwert, da sich ein Abstand zwischen der Mantelfläche und dem Stift, insbesondere im Bereich der Stiftaufnahmenöffnung, mit zunehmendem Winkel vergrößert.

Eine Ausführungsform des Schulterspacers ist dadurch gekennzeichnet, dass die konusartige Stiftaufnahmen eine Mantelfläche aufweisen, welche mit einer Konuslängsachse der Stiftaufnahmen einen Winkel in einem Bereich von 10-30 °, vorzugsweise zwischen 15-25 ° einschließen. Dieser Winkelbereich stellt einen guten Kompromiss zwischen Variabilität der Ausrichtung der Stifte und sicherer und einfacher Befestigungsmöglichkeit der Stifte in den Stiftaufnahmen dar.

Die Stifte können in unterschiedlichen Längen und/oder mit unterschiedlichen Durchmessern vorliegen. Beispielsweise weisen die Stifte eine Länge in einem Bereich von 8-45 mm, vorzugsweise in einem Bereich von 10-40 mm, und einen Durchmesser in einem Bereich von 2-7 mm, vorzugsweise in einem Bereich von 2,5-6,6 mm, auf.

Der Durchmesser der Stifte kann über deren gesamte Länge konstant sein. Der Durchmesser der Stifte kann aber auch über deren Länge variieren. Beispielsweise kann sich der Durchmesser der Stifte von einem Ende zum anderen Ende verjüngen oder die Stifte können an beiden Stiftenden einen geringeren Durchmesser als zwischen den beiden Stiftenden aufweisen. Insbesondere kann es bevorzugt sein, dass sich der Durchmesser der Stifte an dem Ende verjüngt, mit welchem diese in den Stiftaufnahmen befestigt werden, was eine Befestigung erleichtern kann.

Die Stifte können dabei alle eine gleiche Länge und einen gleichen Durchmesser, insbesondere einen gleichen durchschnittlichen Durchmesser, aufweisen.

Eine Ausführungsform des Schulterspacers ist dadurch gekennzeichnet, dass die Stifte einen unterschiedlichen Durchmesser, insbesondere einen unterschiedlichen durchschnittlichen Durchmesser, und/oder eine unterschiedliche Länge aufweisen. Darunter ist nicht zu verstehen, dass alle Stifte zueinander eine unterschiedliche Länge und/oder einen unterschiedlichen Durchmesser aufweisen, also jeder Stift als Unikat vorliegt, sondern dass die Stifte zumindest in zwei unterschiedlichen Längen und/oder mit zwei unterschiedlichen Durchmessern vorliegen. Dies erlaubt eine verbesserte Anpassung des Schulterspacers an im Glenoid des Patienten vorhandene Verankerungslöcher, welche in unterschiedlichen Tiefen und/oder mit unterschiedlichen Durchmessern vorliegen können.

Die Stifte umfassen vorzugsweise, wie die Glenoidkomponente und die Humeruskomponente und aus den gleichen Beweggründen, einen Polymethylmethacrylat-Knochenzement oder bestehen aus einem Polymethylmethacrylat-Knochenzement.

Eine Ausführungsform des Schulterspacers ist dadurch gekennzeichnet, dass der oder die Stifte einen Stiftmetallkern aufweisen. Vorzugsweise liegt der Stiftmetallkern vollständig im Inneren des Stiftes und ist somit vollständig von einer Außenhülle, vorzugsweise aus einem Polymethylmethacrylat-Knochenzement, umgeben. Der Stiftmetallkern verläuft vorzugsweise im Wesentlichen parallel zu der Längsachse des Stiftes und vorzugsweise zumindest über 70 Prozent einer Gesamtlänge des Stiftes. Der Stiftmetallkern dient der Verbesserung der mechanischen Stabilität des Stiftes.

Eine Ausführungsform des Schulterspacers ist dadurch gekennzeichnet, dass die Humeruskomponente einen Humeruskomponentenmetallkern aufweist, um die mechanische Stabilität, analog zu dem bevorzugten Stiftmetallkern, zu verbessern. Vorzugsweise weist die Humeruskomponente einen Schaft auf, welcher im implantierten Zustand des Schulterspacers im Knochenkanal des Humerus des Patienten befestigt ist, und der Humeruskomponentenmetallkern verläuft zumindest anteilig innerhalb des Schaftes.

Ein weiterer Gegenstand der Erfindung betrifft eine Gießform zur Herstellung eines inversen Schulterspacer nach einer der vorhergehend beschriebenen Ausführungsformen der Erfindung, umfassend
eine aus mindestens zwei Teilen bestehende Humerusteilgießform zur Ausbildung der Humeruskomponente,
eine aus mindestens zwei Teilen bestehende Glenoidteilgießform zur Ausbildung der Glenoidkomponente und
eine aus mindestens zwei Teilen bestehende Stiftteilgießform zur Ausbildung des Stiftes oder der Stifte.

Eine Gießform ist eine Hohlform, welche im Wesentlichen eine Außenkontur der einzelnen Bauteile des Schulterspacers umschließen und welche mit einem geeigneten Material, vorzugsweise einem Polymethylmethacrylat-Knochenzementteig, befüllbar ist, um durch Aushärtung des Materials den Schulterspacer bereitzustellen. Vorzugsweise wird das ausgehärtete Material nach dem Aushärten aus der Gießform herausgelöst. Die Gießform ist also vorzugsweise nicht Teil des Schulterspacers.

Die Gießform umfasst mehrere Teilgießformen, vorzugsweise mehrere voneinander separate Teilgießformen, insbesondere eine Humerusteilgießform zur Herstellung der Humeruskomponente des Schulterspacers, eine Glenoidteilgießform zur Herstellung der Glenoidkomponente des Schulterspacers sowie mindestens eine Stiftteilgießform zur Herstellung des oder der Stifte des Schulterspacers.

Jede der Teilgießformen besteht aus mindestens zwei Teilen, so dass die entsprechenden Komponenten und Stifte nach ihrer Herstellung leicht aus den Teilgießformen herausgelöst werden können. Vorzugsweise besteht jede der Teilgießformen aus genau zwei Teilen, wobei die beiden Teilgießformen zumindest spiegelsymmetrisch zueinander aufgebaut sind, so dass jedes dieser Teile eine im Wesentlichen spiegelsymmetrische Längsschnitthälfte der entsprechenden Komponente nachbildet.

Eine Ausführungsform der Gießform ist dadurch gekennzeichnet, dass sie eine Vielzahl, also mindestens zwei, an Stiftteilgießformen mit einem unterschiedlichen Stiftgießformdurchmesser und/oder einer unterschiedlichen Stiftgießformlänge umfasst. Der Stiftteilgießformdurchmesser und die Stiftteilgießformlänge bezieht sich jeweils auf eine Hohlraumlänge beziehungsweise einen Hohlraumdurchmesser der Stiftteilgießform, so dass mit diesen Stiftteilgießformen die Herstellung von Stiften mit unterschiedlichen Durchmessern und/oder Längen möglich ist. Vorzugsweise ist die Stiftteilgießform geeignet, gleichzeitig mehrere Stifte, vorzugsweise mit unterschiedlichen Längen und/oder Durchmesser, herzustellen. Beispielsweise umfasst die Stiftteilgießform Hohlräume zur Herstellung von sechs Stiften, wobei drei der sechs Stifte einen geringeren Durchmesser aufweisen als die verbleibenden der sechs Stifte.

Eine Ausführungsform des Verfahrens ist dadurch gekennzeichnet, dass die Humerusteilgießform Befestigungsmittel zum Einlegen, insbesondere zum im Wesentlichen mittigen Einlegen, eines Humeruskomponentenmetallkerns umfasst. In einer Ausgestaltungsform weist die Humerusteilgießform Ausstülpungen an ihrer Innenwand auf, in welche ein Humeruskomponentenmetallkern einlegbar ist.

In einer weiteren Ausführungsform weist ein Humeruskomponentenmetallkern Ausstülpungen auf, welche mit einer Innenwand der Humerusteilgießform zusammenwirken können, um ein Einlegen an des Humeruskomponentenmetallkerns an einer gewünschten Stelle innerhalb der Humerusteilgießform zu ermöglichen.

Die Gießform, beziehungsweise jeder der Teilgießformen, kann aus unterschiedlichen Materialien oder Materialkombinationen beschaffen sein.

Eine Ausführungsform der Gießform ist dadurch gekennzeichnet, dass die Teilgießformen aus einem transluzenten Polymer bestehen. Dies erlaubt eine optische Kontrolle eines ordnungsgemäßen Befüllens der Teilgießformen mit einem geeigneten Material, insbesondere einem Polymethylmethacrylat-Knochenzementteig, im Zuge der Herstellung des Schulterspacers.

Die einzelnen Teile der Teilgießformen können auf unterschiedliche Weisen miteinander verbindbar sein, um die Teilgießform bereitzustellen. Beispielsweise können die einzelnen Teile mit Schrauben miteinander verbindbar sein.

Um ein möglichst einfaches und insbesondere auch reversibles Verbinden der einzelnen Teile der Teilgießformen zu ermöglichen, ist eine Ausführungsform der Gießform dadurch gekennzeichnet, dass die einzelnen Teile der Teilgießformen durch Rastelemente reversibel lösbar miteinander verbindbar sind. Dies erlaubt ebenso ein schnelles Lösen der Teile nach dem Aushärten der einzelnen Bauteile des Schulterspacers.

Ein weiterer Gegenstand der Erfindung betrifft ein Kit zur Herstellung eines inversen Schulterspacers nach einer der vorhergehenden Ausführungsformen der Erfindung umfassend eine Gießform nach einer der vorhergehenden Ausführungsformen der Erfindung sowie ein Knochenzementpulver und eine Monomerflüssigkeit zum Bereitstellen eines Polymethylmethacrylat-Knochenzementteigs. Das Kit erlaubt die Herstellung eines erfindungsmäßen Schulterspacers umfassend einen Polymethylmethacrylat-Knochenzement oder bestehend aus einem Polymethylmethacrylat-Knochenzement.

In einer Ausführungsform des Kits umfasst dieses zumindest noch einen Humeruskomponentenmetallkern und/oder ein oder mehrere Stiftmetallkerne zur Herstellung von mit Metallkernen verstärkten Humeruskomponenten und/oder Stiften.

Ein weiterer Gegenstand der Erfindung betrifft ein Verfahren zur Herstellung eines inversen Schulterspacers nach einer der vorhergehenden Ausführungsformen der Erfindung mittels eines Kits nach einer der vorhergehenden Ausführungsformen der Erfindung, umfassend die folgenden Verfahrensschritte:
a. Bereitstellen eines Polymethylmethacrylat-Knochenzementteigs durch Anmischen des Knochenzementpulvers mit der Monomerflüssigkeit;
b. Befüllen der Humerusteilgießform, der Glenoidteilgießform und der Stiftteilgießform mit dem Polymethylmethacrylat-Knochenzementteig;
c. Aushärten des Polymethylmethacrylat-Knochenzementteigs unter Bereitstellung der Humeruskomponente, der Glenoidkomponente und des Stiftes.

In einem Verfahrensschritt a. wird ein Polymethylmethacrylat-Knochenzementteig bereitgestellt. Das Bereitstellen kann durch einfaches Anmischen des Knochenzementpulvers mit der Monomerflüssigkeit, beispielsweise mittels Umrührens mit einem Spatel, bewerkstelligt werden. Das Anmischen des Polymethylmethacrylat-Knochenzementteigs kann auch mittels einer sogenannten Pre-Pack-Mischvorrichtung bewerkstelligt werden. Derartige Vorrichtungen umfassen steril verpackt das Knochenzementpulver und die Monomerflüssigkeit und erlauben ein vereinfachtes, anwendungssicheres und steriles Bereitstellen des Polymethylmethacrylat-Knochenzementteigs. Derartige Vorrichtung sind beispielsweise bei Heraeus Medical GmbH, Deutschland unter der Bezeichnung "PALACOS^{®} R+G pro - All-in-One Fixation System^{™}" erhältlich.

Bei Bedarf können dem Polymethylmethacrylat-Knochenzementteig beim Anmischen ein oder mehrere Antibiotika oder sonstige pharmazeutischen Wirkstoffe zugesetzt werden, falls diese nicht bereist im Knochenzementpulver und/oder der Monomerflüssigkeit zugesetzt waren.

In einem Verfahrensschritt b. erfolgt ein Befüllen der Teilgießformen mit dem Polymethylmethacrylat-Knochenzementteig. Vorzugsweise weisen die Teilgießformen dazu eine Einfüllöffnung auf, in welche der Polymethylmethacrylat-Knochenzementteig eingefüllt wird. Vorzugsweise erfolgt das Einfüllen aus durch Austragen des Polymethylmethacrylat-Knochenzementteigs aus einer Pre-Pack-Mischvorrichtung.

In einem Verfahrensschritt c. erfolgt das Aushärten des Polymethylmethacrylat-Knochenzementteigs. Im Anschluss können die fertiggestellten Bauteile des Schulterspacers aus den Teilgießformen entnommen werden.

Der oder die Stifte können zu unterschiedlichen Zeitpunkten des Herstellungsprozesses mit der Glenoidkomponente, insbesondere in den Stiftaufnahmen der Glenoidkomponente, befestigt werden. Beispielsweise können der oder die Stifte zuerst mit dem Glenoid eines Patienten und dann mit der Glenoidkomponente befestigt werden.

Eine Ausführungsform des Verfahrens ist dadurch gekennzeichnet, dass in einem Verfahrensschritt d. ein Befestigen des Stiftes in einer der Stiftaufnahmen der Glenoidkomponente stattfindet. Vorzugsweise findet die Befestigung des Stiftes zeitlich vor einer Befestigung des Stiftes in einem Verankerungsloch im Glenoid eines Patienten statt. Vorzugsweise wird der Stift unter Zuhilfenahme eines Polymethylmethacrylat-Knochenzementteigs in der Stiftaufnahme befestigt.

Die Anmeldung nennt an einigen Stellen Polymethylmethacrylat-Knochenzemente beziehungsweise Polymethylmethacrylat-Knochenzementteige. Unter einem Polymethylmethacrylat-Knochenzementteig wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Durch Aushärtung wird aus einem Polymethylmethacrylat-Knochenzementteig (PMMA-Knochenzementteig) ein Polymethylmethacrylat-Knochenzement (PMMA-Knochenzement). PMMA-Knochenzemente kommen schon lange in medizinischen Anwendungen zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer flüssigen Ausgangskomponente, insbesondere einer Monomerflüssigkeit, als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzementteig. Dabei wird eine Polymerisation des Monomers durch Radikale eingeleitet. Mit fortschreitender Polymerisation des Monomers erhöht sich die Viskosität des Knochenzementteigs, bis dieser vollständig aushärtet.

Unter einem Knochenzementpulver wird ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulver zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver als hydrophiles Additiv mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin. Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

Die für den inversen Schulterspacer offenbarten Merkmale sind auch für die Gießform, das Kit sowie das Verfahren offenbart und umgekehrt.

### Figuren

Die Erfindung wird im Folgenden durch Figuren weiter beispielhaft illustriert. Die Erfindung ist nicht auf die Figuren beschränkt.

Es zeigen
- Fig. 1: einen schematischen Längsschnitt eines beispielhaften inversen Schulterspacers umfassend eine Glenoidkomponente und eine Humeruskomponente,
- Fig. 2: den Schulterspacer aus Figur 1 in einer perspektivischen Frontansicht,
- Fig. 3: den Schulterspacer aus den Figuren 1 und 2 in einer perspektivischen Seitenansicht,
- Fig. 4: einen vergrößerten Ausschnitt der Glenoidkomponente aus den Figuren 1 bis 3 in einem schematischen Längsschnitt,
- Fig. 5: einen schematischen Längsschnitt einer Glenoidteilgießform einer Gießform zur Herstellung des Schulterspacers aus den Figuren 1 bis 4,
- Fig. 6: einen schematischen Längsschnitt einer Humerusteilgießform einer Gießform zur Herstellung des Schulterspacers aus den Figuren 1 bis 4,
- Fig. 7: einen schematischen Querschnitt einer Stiftteilgießform einer Gießform zur Herstellung des Schulterspacers aus den Figuren 1 bis 4, und
- Fig. 8: eine schematische Aufsicht auf die Stifteilgießform aus Figur 7.

### Beschreibung der Figuren

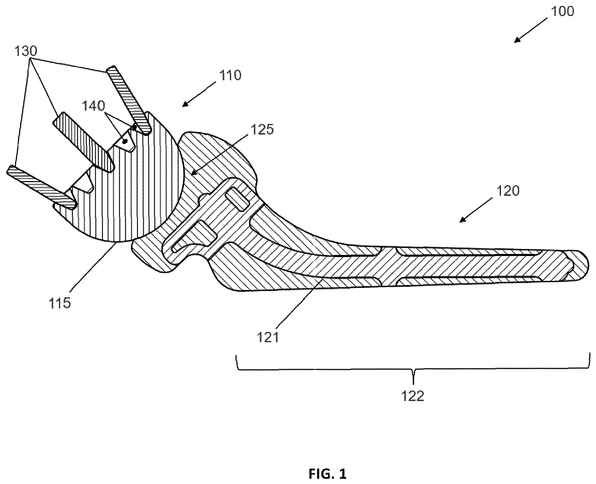

**Figur 1** zeigt einen schematischen Längsschnitt eines beispielhaften inversen Schulterspacers 100. Der Schulterspacer 100 weist eine Glenoidkomponente 110 auf, welche an ihrem distalen Glenoidkomponentenende als eine konvexe Halbkugel 115 ausgebildet ist. Weiterhin weist der Schulterspacer 100 eine Humeruskomponente 120 umfassend eine konkave Kugelaufnahme 125 zur Aufnahme der Halbkugel 115 der Glenoidkomponente 110 auf. Die Halbkugel 115 bildet zusammen mit der Kugelaufnahme 125 in der in Figur 1 gezeigten Position, ebenso wie im implantierten Zustand des Schulterspacers 100 vorgesehen, ein inverses Schultergelenk nach.

An einem der Halbkugel 115 gegenüberliegendem proximalen Glenoidkomponentenende weist die Glenoidkomponente 110 eine Vielzahl an Stiftaufnahmen 140 (lediglich exemplarisch eingezeichnet), insbesondere konusartiger Stiftaufnahmen 140, auf. In ausgewählten Stiftaufnahmen 140 sind Stifte 130 befestigt, welche zur Befestigung des Schulterspacers 100 in Verankerungslöchern im Glenoid eines Patienten dienen. Die Anzahl, Lage und Ausrichtung der Stifte 130 richtet sich vorzugsweise nach bereits von einer primären Schulterprothese herrührenden vorhandenen Verankerungslöchern im Glenoid des Patienten. In der gezeigten Ausführungsform sind die Stifte 130 mittels eines Polymethylmethacrylat-Knochenzement in den Stiftaufnahmen 140 befestigt (nicht eingezeichnet).

Zur Verbesserung der mechanischen Stabilität des Schulterspacers 100, insbesondere der Humeruskomponente 120, ist in der Humeruskomponente 120 ein Humeruskomponentenmetallstift 121 eingebettet, welcher sich über einen Großteil der Länge der Humeruskomponente 120, insbesondere eines Schafts 122 der Humeruskomponente, erstreckt. Der Schaft 122 dient einer Befestigung der Humeruskomponente 120 in einem Knochenkanal des Humerus des Patienten.

In der gezeigten Ausführungsform ist der Schulterspacer 100 aus einem Polymethylmethacryal-Knochenzement gefertigt, wobei der Humeruskomponentenmetallkern 121 aus Edelstahl besteht.

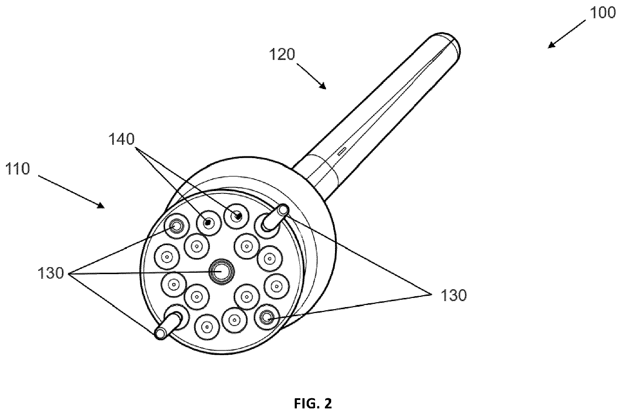

**Figur 2** zeigt den Schulterspacer 100 aus Figur 1 in einer perspektivischen Frontaufsicht auf die Glenoidkomponente 100. In der gezeigten Ansicht ist erkennbar, dass die Glenoidkomponente 110 insgesamt siebzehn Stiftaufnahmen 140 aufweist (lediglich exemplarisch eingezeichnet), welche sich im Wesentlichen über das gesamte proximale Glenoidkomponentenende verteilen. Ebenso ist erkennbar, dass der Schulterspacer 100 insgesamt fünf Stifte 130 aufweist, wobei die Stifte 130 zwei unterschiedliche Durchmesser aufweisen. Der zentrale Stift 130 besitzt dabei einen größeren Durchmesser als die am Rand der Glenoidkomponente 110 befestigten vier Stifte 130. Der Durchmesser der Stifte 130 richtet sich nach den Verankerungslöchern des Patienten und ist hier lediglich exemplarisch in der gezeigten Konfiguration dargestellt.

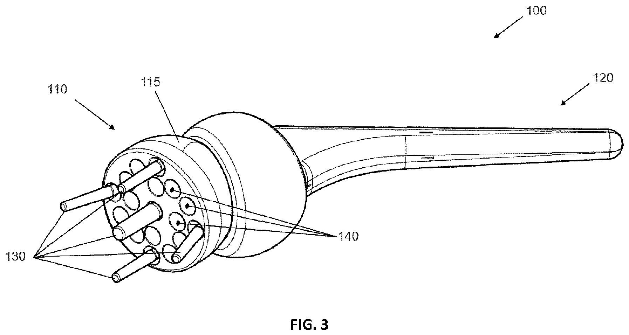

**Figur 3** zeigt den Schulterspacer 100 aus den Figuren 1 und 2 in einer perspektivischen Seitenansicht zur besseren Übersicht. Um Wiederholungen zu vermeiden, wird zur Beschreibung der einzelnen Merkmale auf die Beschreibung der vorhergehenden Figuren verwiesen.

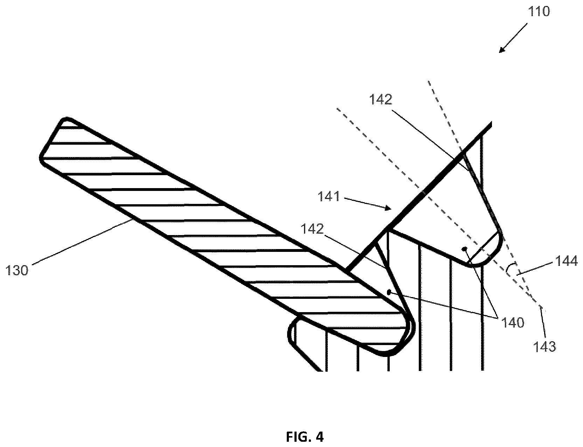

**Figur 4** zeigt einen vergrößerten Ausschnitt der Glenoidkomponente 110 aus den Figuren 1 bis 3 in einem schematischen Längsschnitt. Der Ausschnitt zeigt insbesondere das proximale Glenoidkomponentenende mit zwei Stiftaufnahmen 140, wobei in einer der zwei Stiftaufnahmen 140 ein Stift 130 befestigt ist. Die Stiftaufnahmen 140 (alle Stiftaufnahmen, nicht nur die gezeigten zwei) verfügen über eine Stiftaufnahmenöffnung 141, welche zum Einbringen eines Stiftes 130 in die Stiftaufnahmen 140 dient. In der gezeigten Ausführungsform sind die Stiftaufnahmen 140 konusartig ausgestaltet. Dies bedeutet, dass eine Mantelfläche 142, in anderen Worten die seitliche Wandung, der Stiftaufnahmen 140 mit einer Längsachse 143 der Stiftaufnahmen 140 einen Winkel 144 einschließt (lediglich exemplarisch an der Stiftaufnahme 140 ohne Stift 130 eingezeichnet) und sich somit ein Innenumfang der Stiftaufnahmen 140 von der Stiftaufnahmenöffnung 141 in Richtung der Halbkugel 115 (vgl. Figur 1) verjüngt. In der gezeigten Ausführungsform beträgt der Winkel 144 20 °. Die Stifte 130 weisen hingegen einen geringeren Durchmesser als die Stiftaufnahme 140, zumindest an den Stiftaufnahmenöffnungen 141 und bis zu einer bestimmten Tiefe der Stiftaufnahmen 140, auf. Dies erlaubt eine Auslenkung einer Längsachse der Stifte 130 von der Längsachse der Stiftaufnahmen und so mehr Flexibilität bei der Befestigung der Stifte 130 in den Stiftaufnahmen 140, wie an der in Figur 4 mit einem Stift 130 befüllten gezeigten Stiftaufnahme 140.

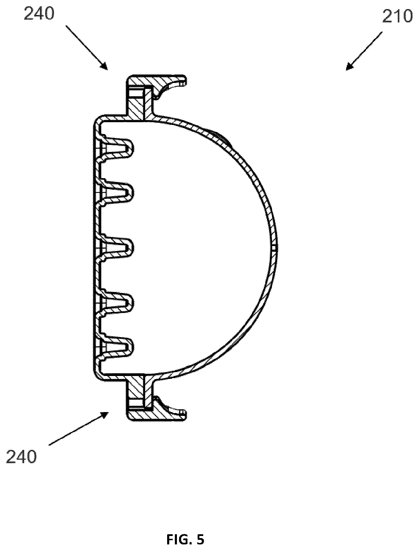

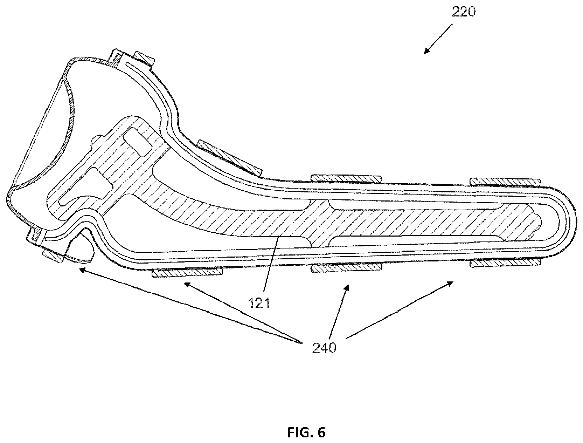

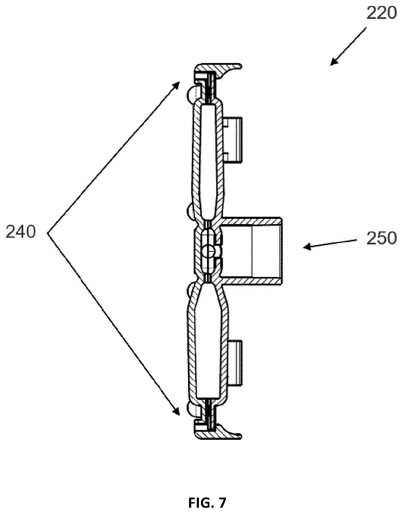

**Figur 5** zeigt eine Glenoidteilgießform 210 zur Herstellung der Glenoidkomponente 110 des Schulterspacers 100 aus den Figuren 1 bis 4 in einem schematischen Längsschnitt. Die Glenoidteilgießform 210 bildet zusammen mit einer Humerusteilgießform 220 (vgl. Fig. 6) und einer Stiftteilgießform 230 (vgl. Fig. 7) eine Gießform zur Herstellung des Schulterspacers 100 aus den Figuren 1 bis 4.

Die Glenoidteilgießform 210 ist in der gezeigten Ausführungsform aus zwei Teilen aufgebaut, welche mit Rastelementen 240 reversibel lösbar miteinander verbunden sind. Dabei bildet eines dieser Teile im Wesentlichen die Halbkugel 115 (vgl. Fig. 1) und das andere im Wesentlichen das proximale Glenoidkomponentenende mit den Stiftaufnahmen 140 (vgl. Fig. 1) nach. Die Glenoidteilgießform 210 kann mit einem geeigneten Material, vorzugsweise einem Polymethylmethacrylat-Knochenzementteig, befüllt werden, welches dann unter Aushärtung die Glenoidkomponente 110 des Schulterspacers 100 bereitstellt. Die Rastelemente 240 erlauben ein einfaches und schnelles Öffnen der Glenoidteilgießform 210 zur Entnahme der Glenoidkomponente 110.

**Figur 6** zeigt mit der Humerusteilgießform 220 eine zweite Teilgießform der Gießform zur Herstellung des Schulterspacers 100, insbesondere zur Herstellung der Humeruskomponente 120, aus den Figuren 1 bis 4 in einem schematischen Längsschnitt. Die Humerusteilgießform 220 umfasst drei Teile, wobei ein Teil im Wesentlichen die Kugelaufnahme 125 (vgl. Fig. 1) nachbildet. Die beiden restlichen Teile der Humerusteilgießform 220 formen im Wesentlichen den Schaft 122 (vgl. Fig. 1) aus, wobei alle Teile durch Rastelemente 240 (lediglich exemplarisch eingezeichnet) miteinander verbunden sind. Innerhalb der Humerusteilgießform 220 ist der Humeruskomponentenmetallkern 121 aufgenommen, um mit einem geeigneten Material, vorzugsweise einem Polymethylmethacrylat-Knochenzementteig, ummantelt zu werden, welches dann unter Aushärtung die Humeruskomponente 120 des Schulterspacers 100 bereitstellt. Die Rastelemente 240 erlauben ein einfaches und schnelles Öffnen der Humerusteilgießform 220 zur Entnahme der Humeruskomponente 120.

**Figur 7** zeigt mit der Stiftteilgießform 230 eine dritte Teilgießform der Gießform zur Herstellung der Schulterspacers 100, insbesondere zur Herstellung der Stifte 130, aus den Figuren 1 bis 4 in einem schematischen Querschnitt. Die Stiftteilgießform 230 umfasst zwei Teile, welche durch Rastelemente 240 reversibel miteinander verbunden sind. Die beiden Teile formen zusammengenommen die Stifte aus. Eine der Teile umfasst eine Einfüllöffnung 250 zum Befüllen der Stiftteilgießform 230 mit einem geeigneten Material, insbesondere einem Polymethylmethacrylat-Knochenzementteig, zur Herstellung der Stifte 130 (vgl. Fig. 1). Jede der Teilgießformen 210, 220, 230 umfasst solch eine Einfüllöffnung 250, auch wenn diese in den Figuren 5 und 6 nicht eingezeichnet ist.

Die Stiftteilgießform 230 dient zur Herstellung von Stiften 130 mit unterschiedlichen Durchmessern, insbesondere unterschiedlichen durchschnittlichen Durchmessern. Im oberen gezeigten Teil der Stiftteilgießform 230 können Stifte mit kleinerem, im unteren Teil mit größerem Durchmesser hergestellt werden.

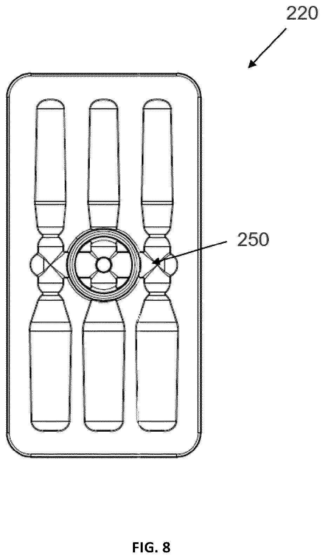

**Figur 8** zeigt die Stiftteilgießform 230 aus Figur 7 in einer schematischen Aufsicht. In Figur 8 ist zu erkennen, dass die Stiftteilgießform 230 zur gleichzeitigen Herstellung von insgesamt sechs Stiften, jeweils drei Stiften mit kleinerem und drei Stiften mit größerem Durchmesser, geeignet ist.

### Bezugszeichen

- 100: inverser Schulterspacer
- 110: Glenoidkomponente
- 115: konvexe Halbkugel
- 120: Humeruskomponente
- 121: Humeruskomponentenmetallkern
- 122: Schaft
- 125: konkave Kugelaufnahme
- 130: Stift
- 140: Stiftaufnahme
- 141: Stiftaufnahmenöffnung
- 142: Mantelfläche
- 143: Konuslängsachse
- 144: Winkel
- 210: Glenoidteilgießform
- 220: Humerusteilgießform
- 230: Stiftteilgießform
- 240: Rastelemente
- 250: Einfüllöffnung

## Patentansprüche

1. Inverser Schulterspacer (100) zum zeitweisen Ersetzen eines künstlichen Schultergelenks, umfassend eine Humeruskomponente (120),
**dadurch gekennzeichnet, dass**
der Schulterspacer (100) eine Glenoidkomponente (110) umfasst, welche an einem distalen Glenoidkomponentenende als eine konvexe Halbkugel (115) ausgebildet ist,
und dass die Humeruskomponente (120) an einem proximalen Humeruskomponentenende eine konkave Kugelaufnahme (125) zur Aufnahme der Halbkugel (115) der Glenoidkomponente (110) aufweist, wobei die Halbkugel (115) mit der Kugelaufnahme (125) ein inverses Schultergelenk nachbildet oder nachbilden kann,
und dass der Schulterspacer (100) einen oder mehrere Stifte (130) aufweist, welche jeweils mit einem Stiftende mit einer von einer Vielzahl an konkaven Stiftaufnahmen (140) an einem proximalen Glenoidkomponentenende der Glenoidkomponente (110) verbunden oder verbindbar sind, so dass der Schulterspacer (100) über den oder die Stifte (130) in Verankerungslöchern in einem Glenoid eines Patienten befestigbar ist.

2. Inverser Schulterspacer (100) nach Anspruch 1, wobei die Humeruskomponente (120) und die Glenoidkomponente (110) einen Polymethylmethacrylat-Knochenzement umfassen oder aus einem Polymethylmethacrylat-Knochenzement bestehen.

3. Inverser Schulterspacer (100) nach Anspruch 1 oder 2, wobei die Stiftaufnahmen (140) konusartig ausgebildet sind, wobei sich ein Innenumfang der Stiftaufnahmen (140) von einer Stiftaufnahmenöffnung (141) in Richtung des distalen Glenoidkomponentenendes verjüngt.

4. Inverser Schulterspacer (100) nach Anspruch 3, wobei die konusartigen Stiftaufnahmen (140) eine Mantelfläche (142) aufweisen, welche mit einer Konuslängsachse (143) der Stiftaufnahme einen Winkel (144) in einem Bereich von 10-30 ° einschließt.

5. Inverser Schulterspacer (100) nach einem der vorhergehenden Ansprüche, wobei die Stifte (130) einen unterschiedlichen Durchmesser und/oder eine unterschiedliche Länge aufweisen.

6. Inverser Schulterspacer (100) nach einem der vorhergehenden Ansprüche, wobei der Stift oder die Stifte (130) einen Stiftmetallkern aufweisen.

7. Inverser Schulterspacer (100) nach einem der vorhergehenden Ansprüche, wobei die Humeruskomponente (120) einen Humeruskomponentenmetallkern (121) aufweist.

8. Gießform zur Herstellung eines inversen Schulterspacers (100) nach einem der Ansprüche 1 bis 7, umfassend
eine aus mindestens zwei Teilen bestehende Humerusteilgießform (220) zur Ausbildung der Humeruskomponente (120),
eine aus mindestens zwei Teilen bestehende Glenoidteilgießform (210) zur Ausbildung der Glenoidkomponente (110) und
eine aus mindestens zwei Teilen bestehende Stiftteilgießform (230) zur Ausbildung des Stiftes (130) oder der Stifte (130).

9. Gießform nach Anspruch 8 umfassend eine Vielzahl an Stiftteilgießformen (230) mit einem unterschiedlichen Stiftteilgießformdurchmesser und/oder einer unterschiedlichen Stiftteilgießformlänge.

10. Gießform nach Anspruch 8 oder 9, wobei die Humerusteilgießform Befestigungsmittel zum Einlegen eines Humeruskomponentenmetallkerns umfasst.

11. Gießform nach einem der Ansprüche 8 bis 10, wobei die Teilgießformen aus einem transluzenten Polymer bestehen.

12. Gießform nach einem der Ansprüche 8 bis 11, wobei die einzelnen Teile der Teilgießformen (210, 220, 230) durch Rastelemente (240) reversibel lösbar miteinander verbindbar sind.

13. Kit zur Herstellung eines inversen Schulterspacers (100) nach einem der Ansprüche 1 bis 7 umfassend eine Gießform nach einem der Ansprüche 8 bis 12 sowie ein Knochenzementpulver und eine Monomerflüssigkeit zum Bereitstellen eines Polymethylmethacrylat-Knochenzementteigs.

14. Verfahren zur Herstellung eines inversen Schulterspacers (100) nach einem der Ansprüche 1 bis 7 mittels eines Kits nach Anspruch 13, umfassend die folgenden Verfahrensschritte:
a. Bereitstellen eines Polymethylmethacrylat-Knochenzementteigs durch Anmischen des Knochenzementpulvers mit der Monomerflüssigkeit;
b. Befüllen der Humerusteilgießform (220), der Glenoidteilgießform (210) und der Stiftteilgießform (230) mit dem Polymethylmethacrylat-Knochenzementteig;
c. Aushärten des Polymethylmethacrylat-Knochenzementteigs unter Bereitstellung der Humeruskomponente (120), der Glenoidkomponente (110) und des Stiftes (130).

15. Verfahren nach Anspruch 14, wobei in einem Verfahrensschritt d. ein Befestigen des Stiftes (130) in einer der Stiftaufnahmen (140) der Glenoidkomponente (110) stattfindet.

## Claims

1. An inverse shoulder spacer (100) for temporarily replacing an artificial shoulder joint, comprising a humeral component (120),
**characterized in that**
the shoulder spacer (100) comprises a glenoid component (110), which is designed as a convex half-ball (115) at a distal glenoid component end,
and **in that** the humeral component (120) at a proximal end of the humeral component comprises a concave ball receptacle (125) for receiving the half-ball (115) of the glenoid component (110), the half-ball (115) along with the ball receptacle (125) simulating or being able to simulate an inverse shoulder joint,
and **in that** the shoulder spacer (100) comprises one or more pins (130), which are each connected or connectable, with a relevant pin end, to one of a plurality of concave pin receptacles (140) at a proximal end of the glenoid component (110), such that the shoulder spacer (100) can be fastened via the one or more pins (130) in anchoring holes in a glenoid of a patient.

2. The inverse shoulder spacer (100) according to claim 1, wherein the humeral component (120) and the glenoid component (110) comprise a polymethyl methacrylate bone cement or consist of a polymethyl methacrylate bone cement.

3. The inverse shoulder spacer (100) according to claim 1 or 2, wherein the pin receptacles (140) are conical, wherein an inner circumference of the pin receptacles (140) tapers from a pin receptacle opening (141) toward the distal end of the glenoid component.

4. The inverse shoulder spacer (100) according to claim 3, wherein the conical pin receptacles (140) comprise a lateral surface (142) that encloses an angle (144) in a range of 10-30° with a cone longitudinal axis (143) of the pin receptacle.

5. The inverse shoulder spacer (100) according to any of the preceding claims, wherein the pins (130) have a different diameter and/or a different length.

6. The inverse shoulder spacer (100) according to any of the preceding claims, wherein the pin or pins (130) comprise a metal pin core.

7. The inverse shoulder spacer (100) according to any of the preceding claims, wherein the humeral component (120) comprises a metal humeral component core (121).

8. A mold for producing an inverse shoulder spacer (100) according to any of claims 1 to 7, comprising
a humeral part mold (220), consisting of at least two parts, for forming the humeral component (120),
a glenoid part mold (210), consisting of at least two parts, for forming the glenoid component (110), and
a pin part mold (230), consisting of at least two parts, for forming the pin (130) or the pins (130).

9. The mold according to claim 8, comprising a plurality of pin part molds (230) having a different pin part mold diameter and/or a different pin part mold length.

10. The mold according to claim 8 or 9, wherein the humeral part mold comprises fastening means for inserting a metal humeral component core.

11. The mold according to any of claims 8 to 10, wherein the part molds consist of a translucent polymer.

12. The mold according to any of claims 8 to 11, wherein the individual parts of the part molds (210, 220, 230) can be reversibly detachably connected to one another by locking elements (240).

13. A kit for producing an inverse shoulder spacer (100) according to any of claims 1 to 7, comprising a mold according to any of claims 8 to 12 and a bone cement powder and a monomer liquid for providing a polymethyl methacrylate bone cement paste.

14. A method for producing an inverse shoulder spacer (100) according to any of claims 1 to 7 by means of a kit according to claim 13, comprising the following method steps:
a. providing a polymethyl methacrylate bone cement paste by mixing the bone cement powder with the monomer liquid;
b. filling the humeral part mold (220), the glenoid part mold (210) and the pin part mold (230) with the polymethyl methacrylate bone cement paste;
c. curing the polymethyl methacrylate bone cement paste to provide the humeral component (120), the glenoid component (110), and the pin (130).

15. The method according to claim 14, wherein, in a method step d., the pin (130) is fastened in one of the pin receptacles (140) of the glenoid component (110).

## Revendications

1. Écarteur d'épaule (100) inversé permettant de remplacer temporairement une articulation d'épaule artificielle, comprenant un composant pour humérus (120),
**caractérisé en ce que**
l'écarteur d'épaule (100) comprend un composant pour cavité glénoïde (110) qui est réalisé comme une demi-sphère (115) convexe à une extrémité distale de composant pour cavité glénoïde,
**et en ce que** le composant pour humérus (120) présente, à une extrémité proximale de composant pour humérus, un logement pour sphère (125) concave destiné à recevoir la demi-sphère (115) du composant pour cavité glénoïde (110), dans lequel la demi-sphère (115) reproduit ou peut reproduire avec le logement pour sphère (125) une articulation d'épaule inversée,
**et en ce que** l'écarteur d'épaule (100) présente une ou plusieurs broches (130) qui sont respectivement reliées ou peuvent être respectivement reliées par une extrémité de broche à un logement pour broche d'une pluralité de logements pour broches (140) concaves à une extrémité proximale de composant pour cavité glénoïde du composant pour cavité glénoïde (110), de sorte que l'écarteur d'épaule (100) peut être fixé par l'intermédiaire de la ou des broches (130) dans des trous d'ancrage dans une cavité glénoïde d'un patient.

2. Écarteur d'épaule (100) inversé selon la revendication 1, dans lequel le composant pour humérus (120) et le composant pour cavité glénoïde (110) comprennent un ciment osseux en polyméthacrylate de méthyle ou sont constitués d'un ciment osseux en polyméthacrylate de méthyle.

3. Écarteur d'épaule (100) inversé selon la revendication 1 ou 2, dans lequel les logements pour broches (140) sont réalisés sous forme conique, dans lequel une périphérie interne des logements pour broches (140) se rétrécissent depuis une ouverture de logements pour broches (141) en direction de l'extrémité distale de composant pour cavité glénoïde.

4. Écarteur d'épaule (100) inversé selon la revendication 3, dans lequel les logements pour broches (140) coniques présentent une surface d'enveloppe (142) qui forme un angle (144) compris dans une plage allant de 10 à 30° avec un axe longitudinal conique (143) du logement pour broche.

5. Écarteur d'épaule (100) inversé selon l'une des revendications précédentes, dans lequel les broches (130) présentent des diamètres différents et/ou des longueurs différentes.

6. Écarteur d'épaule (100) inversé selon l'une des revendications précédentes, dans lequel la broche ou les broches (130) présentent un noyau métallique de broche.

7. Écarteur d'épaule (100) inversé selon l'une des revendications précédentes, dans lequel le composant pour humérus (120) présente un noyau métallique de composant pour humérus (121).

8. Moule pour la fabrication d'un écarteur d'épaule (100) inversé selon l'une des revendications 1 à 7, comprenant
un moule partiel pour humérus (220) constitué d'au moins deux parties pour la formation du composant pour humérus (120),
un moule partiel pour cavité glénoïde (210) constitué d'au moins deux parties pour la formation du composant pour cavité glénoïde (110) et
un moule partiel de broche (230) constitué d'au moins deux parties pour la formation de la broche (130) ou des broches (130).

9. Moule selon la revendication 8, comprenant une pluralité de moules partiels pour broches (230) comportant des diamètres de moules partiels pour broches différents et/ou des longueurs de moules partiels pour broches différentes.

10. Moule selon la revendication 8 ou 9, dans lequel le moule partiel pour humérus comprend des moyens de fixation pour l'insertion d'un noyau métallique de composant pour humérus.

11. Moule selon l'une des revendications 8 à 10, dans lequel les moules partiels sont constitués d'un polymère translucide.

12. Moule selon l'une des revendications 8 à 11, dans lequel les différentes parties des moules partiels (210, 220, 230) peuvent être reliées entre elles de manière réversible et amovible par des éléments d'encliquetage (240).

13. Kit pour la fabrication d'un écarteur d'épaule (100) inversé selon l'une des revendications 1 à 7, comprenant un moule selon l'une des revendications 8 à 12, ainsi qu'une poudre de ciment osseux et un liquide monomère pour la fourniture d'une pâte de ciment osseux en polyméthacrylate de méthyle.

14. Procédé pour la fabrication d'un écarteur d'épaule (100) inversé selon l'une des revendications 1 à 7 au moyen d'un kit selon la revendication 13, comprenant les étapes de procédé suivantes :
a. fourniture d'une pâte de ciment osseux en polyméthacrylate de méthyle en mélangeant la poudre de ciment osseux avec le liquide monomère ;
b. remplissage du moule partiel pour humérus (220), du moule partiel pour cavité glénoïde (210) et du moule partiel de broche (230) de pâte de ciment osseux en polyméthacrylate de méthyle ;
c. durcissement de la pâte de ciment osseux en polyméthacrylate de méthyle pour fournir le composant pour humérus (120), le composant pour cavité glénoïde (110) et la broche (130).

15. Procédé selon la revendication 14, dans lequel, dans une étape de procédé d., une fixation de la broche (130) dans l'un des logements pour broches (140) du composant pour cavité glénoïde (110) est effectuée.
